# DEMANDE DE BREVET EUROPEEN

(11) **EP 3 248 540 A1**
(43) Date de publication de la demande: **29.11.2017**
(21) Numéro de dépôt: 17171886.9
(22) Date de dépôt: 19.05.2017
(51) Int. Cl.: A61B 5/021, A61B 5/022, A61B 5/0235

(54) **SYSTEME DE SPHYGMOMANOMETRE**

(30) Priorité: 19.05.2016 FR 1654453
(71) Demandeur: Spengler SAS, 36100 Issoudun (FR)
(72) Inventeur: PESSEY, Daniel, 13100 AIX-EN-PROVENCE (FR)
(74) Mandataire: Roman, Alexis

(57) **Abrégé**

L'invention concerne un système de sphygmomanomètre (1) comprenant:
- un brassard gonflable (2) adapté pour être placé sur le membre d'un patient comportant une poche gonflable (25),
- une poire de gonflage (3) associée à un cordon tubulaire souple (4) pour assurer le gonflage du brassard (2),
- un manomètre pour mesurer la pression artérielle, lequel manomètre est soit associé à la poire de gonflage (3) pour former un mano-poire (MP), soit associé directement au brassard (2) pour former un mano-brassard (MB),
avec des moyens de connexion adaptés pour rendre interchangeable la configuration du système (1), lesquels moyens de connexion sont constitués d'aménagements en forme d'embouts mâles (51, 71) et orifices femelles (6), du type assemblage rapide, qui sont répartis de sorte que ledit cordon soit adapté pour assurer:
- d'une part, une liaison directe entre le mano-poire (MP) et le brassard (2), lorsque le manomètre (5) est connecté sur la poire de gonflage (3) pour former ledit mano-poire (MP),
- et d'autre part, une liaison directe entre la poire de gonflage (3) et le manomètre (5) lorsque ledit manomètre est connecté sur le brassard (2) pour former le mano-brassard (MB).

## Description

### Domaine technique de l'invention.

L'invention a pour objet un système de sphygmomanomètre à configuration interchangeable.

Elle concerne le domaine technique des dispositifs médicaux, et plus particulièrement celui des dispositifs permettant de mesurer la tension artérielle.

### État de la technique.

La mesure de la pression artérielle (ou tension artérielle) est une des mesures les plus courantes effectuées par les médecins. Une mesure rapide et précise est donc bénéfique que ce soit pour le médecin ou pour le patient. La méthode préférée pour effectuer une telle mesure reste l'utilisation d'un tensiomètre (ou sphygmomanomètre).

Un sphygmomanomètre se compose généralement :
- d'un brassard gonflable adapté pour être placé sur le bras d'un patient à examiner et/ou à surveiller, lequel brassard comporte une poche gonflable,
- d'un système de mesure, comme par exemple un manomètre relié au brassard,
- d'une poire gonflable, elle-même reliée au brassard, servant à augmenter la pression d'air dans ledit brassard. Cette poire est généralement équipée d'une soupape permettant de contrôler la pression et de la faire diminuer progressivement de manière à effectuer la mesure.

De nos jours de nombreux sphygmomanomètres sont utilisés. Ils peuvent être manuels ou encore automatiques. Les sphygmomanomètres manuels sont utilisés conjointement avec un stéthoscope, qui permet au médecin de déceler la reprise des battements cardiaques dans l'artère du bras (pression systolique). En lisant à cet instant la valeur indiquée par le manomètre, on obtient la mesure de la pression artérielle maximale. Lorsque la pression du brassard devient inférieure à la pression diastolique, les battements deviennent inaudibles dans le stéthoscope, et la valeur fournie par le manomètre correspond à la pression artérielle minimale.

Il existe de nombreuses causes pouvant amener à une mauvaise mesure de pression artérielle. Le choix du sphygmomanomètre utilisé est donc primordial pour que le diagnostic ne soit pas erroné.

Différents types de sphygmomanomètres ont été développés, principalement le mano-brassard et le mano-poire.

Dans un mano-brassard, le manomètre est directement fixé sur le brassard, rendant la lecture de la valeur de la pression artérielle plus aisée et le système moins lourd à porter, qualité non négligeable pour un médecin effectuant un grand nombre de mesures par jour. Toutefois, ce type de sphygmomanomètre s'avère difficile à placer sur certains types de patients, en particulier les personnes âgées, les enfants, ou encore les nourrissons, pour lesquels l'artère du bras est difficilement décelable.

Dans un mano-poire, le manomètre est associé à la poire de gonflage, un cordon tubulaire souple reliant ledit mano-poire audit brassard pour assurer le gonflage de ce dernier. Ce type de brassard est plus simple à poser qu'un mano-brassard. Aussi, le médecin peut favoriser l'utilisation d'un mano-poire pour les patients pour lesquels l'artère du bras est difficilement décelable.

Le médecin doit donc avoir à sa disposition ces deux types de sphygmomanomètres, à savoir un mano-brassard et un mano-poire, ce qui peut s'avérer couteux. Pour le mano-brassard, il doit avoir à disposition une poire de gonflage et un cordon souple. Pour le mano-poire, il doit avoir à disposition un brassard et un cordon souple. Toutes ces pièces sont relativement encombrantes, ce qui peut être très gênant pour les médecins effectuant des visites à domicile et devant transporter leur matériel.

L'invention vise à remédier à cet état des choses. En particulier, un objectif de l'invention est de proposer un système de sphygmomanomètre qui soit moins coûteux et moins encombrant que le système de l'art antérieur.

Un autre objectif de l'invention est de proposer un système de sphygmomanomètre permettant au médecin d'avoir rapidement tous les types de sphygmomanomètres nécessaires à sa disposition avec un minimum d'encombrement et un minimum de pièces.

### Divulgation de l'invention.

La solution proposée par l'invention est un système de sphygmomanomètre comprenant :
- un brassard gonflable adapté pour être placé sur le membre d'un patient à examiner et/ou à surveiller, lequel brassard comporte une poche gonflable,
- une poire de gonflage associée à un cordon tubulaire souple pour assurer le gonflage du brassard,
- un manomètre pour mesurer la pression artérielle, lequel manomètre est soit associé à la poire de gonflage pour former un mano-poire, soit associé directement au brassard pour former un mano-brassard.

Ce système est remarquable en ce que des moyens de connexion sont adaptés pour rendre interchangeable la configuration du système, lesquels moyens de connexion sont constitués d'aménagements en forme d'embouts mâles et orifices femelles, du type assemblage rapide, qui sont répartis :
- sur le brassard,
- sur le manomètre,
- sur la poire de gonflage,
- le mano-poire,
- sur chaque extrémité du cordon tubulaire, de sorte que ledit cordon soit adapté pour assurer :
   ∘ d'une part, une liaison directe entre le mano-poire et le brassard, lorsque le manomètre est connecté sur la poire de gonflage pour former ledit mano-poire,
   ∘ et d'autre part, une liaison directe entre la poire de gonflage et le manomètre lorsque ledit manomètre est connecté sur le brassard pour former le mano-brassard.

Un tel système de sphygmomanomètre a donc maintenant une configuration interchangeable de sorte qu'il puisse être modifié et/ou adapté aisément en fonction du patient et/ou des besoins du médecin. Le médecin n'a maintenant besoin que d'un seul brassard, qu'il peut soit configurer en mano-brassard, soit connecter à un mano-poire. Cela permet au médecin de pouvoir aisément passer d'un type de sphygmomanomètre à un autre suivant ses besoins, tout en limitant le nombre d'éléments à transporter.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaires :
- Selon une configuration avantageuse de l'invention : le cordon comporte un embout mâle à chaque extrémité ; le brassard comporte un orifice femelle de connexion ; la poire de gonflage comporte un orifice femelle de connexion pour accueillir un embout mâle situé sur une des extrémités du cordon ; le mano-poire comporte un orifice femelle de connexion pour accueillir un embout mâle situé sur une des extrémités du cordon ; le manomètre comporte un embout mâle pour se connecter sur l'orifice femelle de connexion du brassard et un orifice femelle pour accueillir un embout mâle situé sur une des extrémités du cordon.
- L'orifice femelle du brassard est préférentiellement aménagé dans une protubérance, ledit orifice femelle étant en communication fluidique avec la poche gonflable dudit brassard.
- Cette protubérance peut être façonnée en saillie sur une pièce d'interface, laquelle pièce d'interface fait office d'interface entre la poche gonflable et l'orifice femelle du brassard.
- La pièce d'interface peut être soudée sur le brassard.
- L'axe de l'orifice femelle du brassard est avantageusement parallèle à la surface de la pièce d'interface, ledit axe étant perpendiculaire à l'axe général longitudinal dudit brassard.
- Préférentiellement, un aménagement en relief, en forme de semelle, est installé devant l'orifice femelle et dans l'axe de ce dernier, laquelle semelle fait office de renfort et de guide pour la mise en place de l'embout mâle dans ledit orifice femelle.
- Une structure de joint est préférentiellement installée au niveau des embouts mâles, l'entrée des orifices femelles comportant un chemisage en forme de manchon cylindrique, ledit manchon étant adapté à ladite structure de joints.
- Chaque embout mâle comporte avantageusement plusieurs joints toriques espacés longitudinalement.
- Au moins deux des joints toriques peuvent être réalisés dans des matériaux différents.
- Avantageusement, chaque moyen de connexion du cordon tubulaire comporte un embout mâle externe et un embout mâle interne séparés par une collerette, l'embout externe étant muni d'au moins un joint torique d'étanchéité et l'embout interne étant solidarisé avec ledit cordon, lequel embout interne comporte au moins un collet d'accroche coopérant avec la paroi interne dudit cordon tubulaire.
- Les embouts mâles sont préférentiellement en forme de cylindre dont le diamètre externe est ajusté au diamètre interne du manchon cylindrique.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une représentation schématique d'un système de sphygmomanomètre selon l'invention installé sur le bras d'un patient,
- la figure 2 est une représentation schématique d'un moyen de connexion du cordon tubulaire au brassard du système de la figure 1,
- la figure 3 est une représentation schématique des deux extrémités du cordon tubulaire des figures 1 et 2, chacune des extrémités étant pourvue d'un moyen de connexion,
- la figure 4 est une représentation schématique d'un système de sphygmomanomètre selon l'invention, dans une configuration où le brassard est connecté à un mano-poire,
- la figure 5 est une représentation en coupe d'un moyen de connexion constitué d'un aménagement en forme d'embout mâle et d'un orifice femelle,
- la figure 6 est une représentation schématique d'un système de sphygmomanomètre selon l'invention, dans une configuration où le manomètre est associé directement au brassard pour former un mano-brassard,
- la figure 7 est une vue de coté agrandie du système de sphygmomanomètre de la figure 6, illustrant d'une part la connexion du manomètre sur le brassard et d'autre part la connexion du cordon souple sur ledit manomètre,
- la figure 8 est une vue en perspective d'une pièce d'interface utilisée dans l'invention,
- la figure 9 est une vue en coupe de la pièce d'interface de la figure 8,
- la figure 10 est une vue en coupe d'une variante de réalisation de la pièce d'interface de la figure 8.

### Modes préférés de réalisation de l'invention.

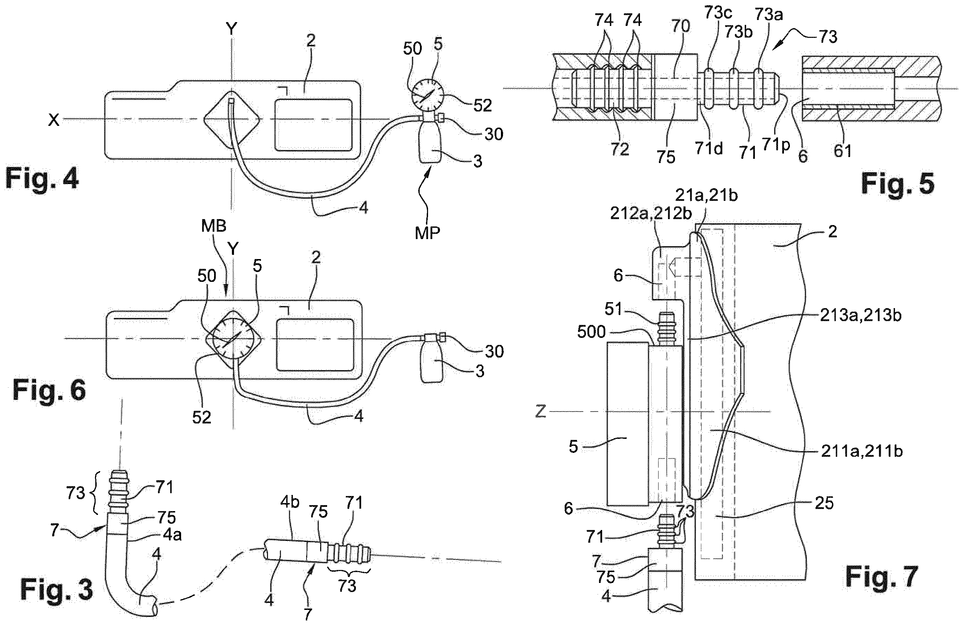

Le système de sphygmomanomètre 1 objet de l'invention est à configuration interchangeable selon les besoins du médecin. Il possède plusieurs éléments pouvant être connectés les uns aux autres de manière à créer différents types de sphygmomanomètres, et en particulier un mano-poire (figure 4) et un mano-brassard (figure 6).

Le système 1 comporte les éléments suivants :
- un brassard gonflable 2 adapté pour être placé sur le bras d'un patient à examiner et/ou à surveiller, lequel brassard comporte une poche gonflable,
- un moyen de gonflage 3, tel qu'une poire de gonflage, associé à un cordon tubulaire souple 4 pour assurer le gonflage du brassard 2,
- un manomètre pour mesurer la pression artérielle systolique et diastolique du patient, lequel manomètre est soit associé à la poire de gonflage 3 pour former un mano-poire MP, soit associé directement au brassard 2 pour former un mano-brassard MB.

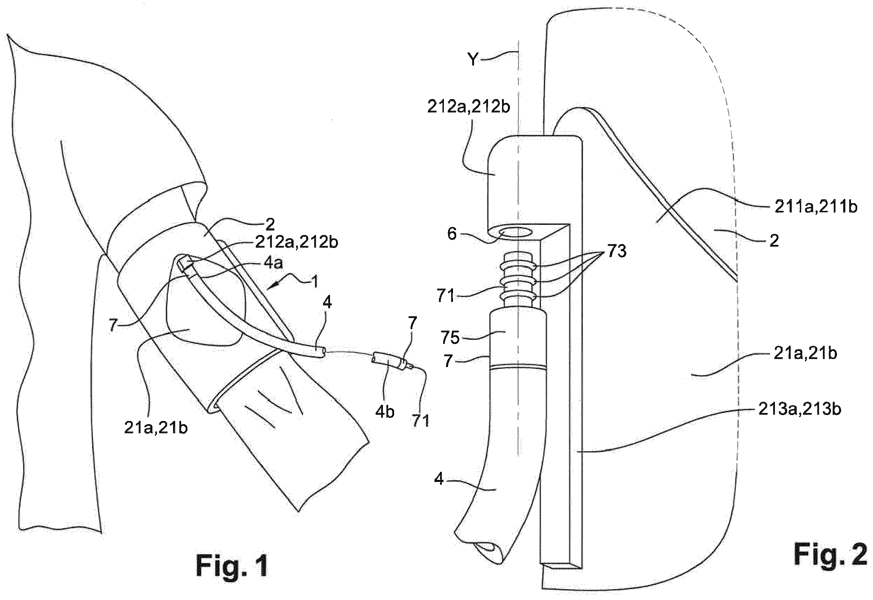

Sur la figure 1, le brassard 2 est installé autour du bras d'un patient, au niveau de l'artère, de manière à effectuer les différentes mesures de tensions artérielles. Le cordon souple 4 permet d'amener l'air pour le gonflage du brassard 2. Lorsqu'il est gonflé, le brassard 2 écrase l'artère sur laquelle il est placé. En écoutant, à l'aide d'un stéthoscope, le bruit émis par le sang lors de son passage dans l'artère, deux valeurs de pression artérielle sont mesurées. Ainsi, lorsque le brassard 2 est suffisamment gonflé pour comprimer l'artère, le sang ne peut plus passer et le médecin ne perçoit pas de bruit. Le brassard 2 se dégonfle ensuite progressivement, permettant de percevoir le bruit des battements cardiaques définissant la pression artérielle systolique (Pression maximale). Ce bruit s'amplifie puis disparaît, la pression mesurée correspondant alors à la pression artérielle diastolique (Pression minimale).

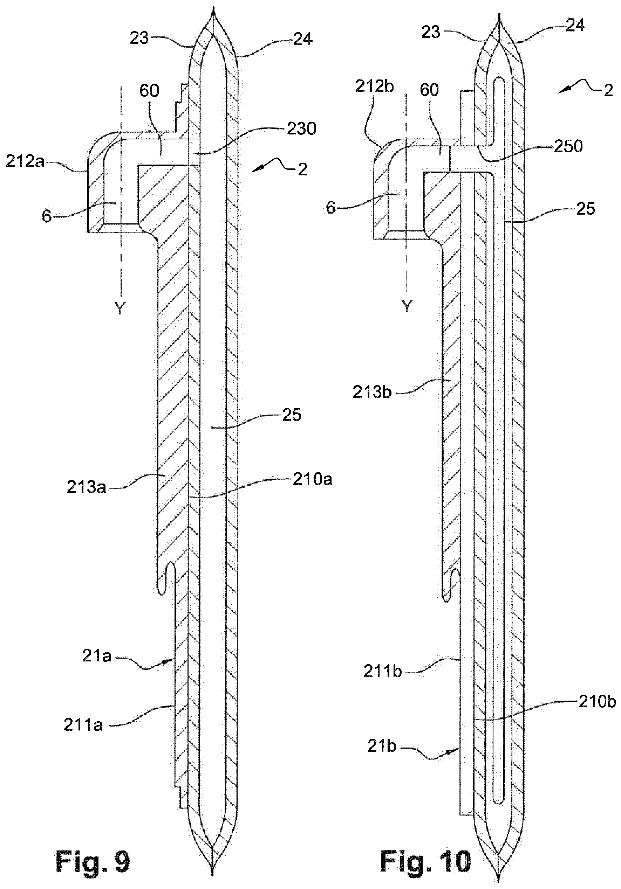

Le brassard 2 peut être de différentes tailles de manière à pouvoir s'adapter à des patients aux morphologies et diamètre de bras différents. Il a par exemple une longueur comprise entre 40 cm et 100 cm, une largeur pouvant varier entre 5 cm et 30 cm. Le brassard 2 est fabriqué de manière conventionnelle et comporte une poche gonflable. Il peut être fabriqué en une seule pièce (figure 9) dont la double paroi 23, 24 délimite la poche gonflable 25, et être réalisé dans un matériau plastique souple imperméable tel qu'un polymère ou un élastomère, comme par exemple le polyéthylène ou encore le polyuréthane. De préférence, il est fabriqué dans un matériau thermoplastique. Le brassard 2 peut également être formé par deux parois 23, 24 en textile non imperméable, tel que le coton, lesquelles parois délimitent une poche dans laquelle est logée la poche gonflable 25 (figure 10), laquelle poche est réalisée dans un matériau plastique souple imperméable tel qu'un polymère ou un élastomère.

Un moyen de gonflage 3 permet d'injecter de l'air dans la poche gonflable 25 du brassard 2 pour le gonfler. Comme illustré sur les figures 4 et 6, ce moyen de gonflage 3 se présente préférentiellement sous la forme d'une poire de gonflage, laquelle poire est du type habituellement utilisé dans les systèmes de sphygmomanomètre connus de l'art antérieur. Il suffit au médecin d'exercer, à la main, des pressions successives sur la poire 3 pour injecter de l'air dans le cordon 4 et gonfler le brassard. La poire 3 est pourvue d'un organe 30 de commande d'une valve ou d'une soupape de mise à l'air libre de la poche gonflable 25 du brassard 2. L'actionnement de cet organe 30 permet notamment de réduire graduellement la pression du brassard 2 gonflé en vue de la détermination des pressions systoliques. Cet organe 30 peut se présenter sous la forme d'une molette ou d'un bouton poussoir.

Le manomètre 5 est du type connu et présente une aiguille 50 coopérant avec une graduation 52 d'un cadran de lecture, pour indiquer la pression artérielle.

Le cordon tubulaire 4 est réalisé dans un matériau souple, comme par exemple un polyéthylène, un polystyrène, ou en tout autre matériau plastique convenant à l'homme du métier. Il a un diamètre pouvant varier de 0.2 cm à 2 cm, et une longueur comprise entre 20 cm et 2 m.

La figure 4 schématise une configuration où le manomètre 5 est associé à la poire de gonflage 3 pour former un mano-poire MP. Ce dispositif unique MP est connu de l'homme du métier. Le mano-poire MP est relié au brassard 2 au moyen du cordon tubulaire 4 permettant à l'air de circuler depuis la poire 3 jusqu'à l'intérieur de la poche dudit brassard.

La figure 6 schématise une autre configuration où le manomètre 5 est associé directement au brassard 2 pour former un mano-brassard MB. La poire de gonflage 3 est raccordée au manomètre 5 par le cordon tubulaire 4. Cette configuration est connue de l'homme du métier.

Conformément à l'invention, des moyens de connexion sont adaptés pour rendre interchangeable la configuration du système 1. Ces moyens de connexion sont constitués d'aménagements en forme d'embouts mâles et orifices femelles, du type assemblage rapide.

Sur les figures 3, 5 et 7, le cordon tubulaire 4 comporte des moyens de connexion 7 situés sur chacune de ses extrémités 4a, 4b. Ces moyens de connexion 7, permettent de raccorder le cordon 4 aux différents éléments constitutifs du système 1.

Sur la figure 3, le cordon 4 comporte un embout mâle 71 à chaque extrémité 4a, 4b. Ces embouts mâles 71 sont adaptés pour s'insérer dans des orifices femelles de connexion 6 complémentaires décrits plus avant dans la description et qui sont situés sur le brassard 2, sur le mano-poire MP, sur la poire de gonflage 3 et/ou sur le manomètre 5.

En se rapportant plus particulièrement à la figure 5, le moyen de connexion 7 est formé de l'embout mâle externe 71 et d'un embout mâle interne 72, ces deux embouts étant reliés par une collerette 75. Pour simplifier sa conception, ce moyen de connexion 7 est avantageusement fabriqué d'un seul tenant. Les embouts 71, 72 et la collerette 75 sont fabriqués dans un matériau rigide, comme par exemple un matériau plastique ou un métal. Les embouts 71, 72 et la collerette 75 sont percés axialement de manière à former un conduit 70 autorisant la circulation d'air.

L'embout mâle externe 71 se présente, de préférence, sous la forme d'un cylindre sur lequel est installée une structure de joint 73. Ce cylindre a par exemple un diamètre externe compris entre 0,15 cm et 2 cm, et une longueur pouvant par exemple varier de 0,5 cm à 2 cm.

Pour assurer une étanchéité à l'air optimale avec les orifices femelles de connexion 6, une structure de joint 73 est installée au niveau de l'embout mâle externe 71. Avantageusement, au moins un joint torique d'étanchéité 73a, et préférentiellement au moins trois joints toriques d'étanchéité 73a, 73b, 73c, sont disposés autour du cylindre de l'embout 71. Ces joints toriques d'étanchéité 73a, 73b, 73c sont espacés longitudinalement, par exemple d'une distance de 0,2 cm à 0,5 mm.

Les joints toriques 73a, 73b, 73c sont préférentiellement réalisés en caoutchouc mais ils peuvent également être réalisés dans un autre matériau, comme par exemple le silicone. Quand l'embout mâle externe 71 comporte plusieurs joints toriques 73a, 73b, 73c, ceux-ci peuvent être réalisés dans des matériaux identiques ou dans des matériaux différents. Sur l'exemple de la figure 5, les joints 73a et 73b situés au niveau de l'extrémité proximale (ou extrémité d'entrée) 71 p de l'embout 71 peuvent être réalisés en caoutchouc et le joint 73c situé au niveau de l'extrémité distale 71 d peut être réalisé en silicone. La demanderesse a constaté qu'une telle configuration offrait les meilleurs résultats en termes d'étanchéité à l'air.

L'embout mâle interne 72 se présente, de préférence, sous la forme d'un cylindre similaire à celui formant l'élément mâle externe 71. Ce cylindre a par exemple un diamètre compris entre 0,15 cm et 2 cm, et une longueur pouvant par exemple varier de 0,5 cm à 2 cm.

L'embout mâle interne 72 est adapté pour s'insérer de manière étanche dans l'extrémité respective 4a, 4b du cordon tubulaire 4 de sorte que le moyen de connexion 7 soit solidarisé à ce dernier. Cet embout mâle interne 72 comporte au moins un collet d'accroche 74 coopérant avec la paroi interne du cordon 4. Ce collet d'accroche 74 permet de l'insérer en force dans le cordon tubulaire 4 et ainsi assurer l'étanchéité à l'air entre le moyen de connexion 7 et ledit cordon. Avantageusement, au moins deux collets 74, et préférentiellement au moins quatre collets d'accroche 74, sont disposés autour du cylindre de l'embout 2. En pratique, le diamètre externe des collets 74 est légèrement supérieur au diamètre interne du cordon 4 (par exemple de 1 mm) pour assurer l'insertion en force de l'embout mâle interne 72 et une déformation plastique de la paroi interne du cordon 4, laquelle déformation assure l'étanchéité à l'air.

La collerette 75 est disposée à l'extérieur du cordon 4 et vient en appui contre l'extrémité respective 4a, 4b de ce dernier. La collerette 75 se présente par exemple sous la forme d'un cylindre dont le diamètre externe correspond au diamètre externe du cordon 4, sa longueur étant par exemple comprise entre 0,5 cm et 1 cm.

Sur la figure 7, le manomètre 5 possède également un embout mâle externe 51 identique à l'embout 71 décrit précédemment. Cet embout mâle externe 51 est situé dans la partie supérieure du manomètre, et fait saille depuis une bordure latérale 500 du cadran, perpendiculairement à l'axe Z dudit cadran.

Les orifices femelles de connexion 6 sont situés sur le brassard 2, sur le mano-poire MP, sur la poire de gonflage 3 et sur le manomètre 5. Sur la figure 5, l'entrée de l'orifice femelle 6 comporte un chemisage en forme de manchon cylindrique 61 adapté à la structure de joints 73. Plus particulièrement, ce manchon cylindrique 61 coopère avec la structure de joints 73 pour assurer une étanchéité à l'air optimale entre un élément mâle 71 et l'orifice femelle de connexion 6 correspondant.

Le manchon cylindrique 61 est fabriqué dans un matériau rigide, comme par exemple un matériau plastique ou un métal. Le cylindre des embouts mâles externes 51, 71 a un diamètre externe ajusté au diamètre interne du manchon cylindrique 61. Par « ajusté », on entend que le diamètre interne du manchon 61 est sensiblement égal au diamètre externe du cylindre formant l'embout mâle externe 51, 71, et préférentiellement légèrement supérieur (par exemple de 0,5 mm) pour éviter le déchaussement des joints 73 lors de l'insertion dudit embout 71. Le manchon 61 a par exemple un diamètre interne compris entre 0,15 cm et 2 cm, et une longueur correspondant à celle du cylindre formant l'embout mâle externe 51, 71. Ainsi, lorsque l'embout mâle externe 51, 71 est inséré axialement dans l'orifice femelle 6, les joints toriques 73a, 73b, 73c viennent en appui contre la surface interne du manchon cylindrique 61, assurant de fait une connexion étanche entre ledit embout 51, 71 et ledit orifice 6.

Le diamètre externe du manchon 61 est tel qu'il soit inséré en force dans l'orifice femelle 6 correspondant, assurant ainsi une étanchéité à l'air satisfaisante entre ledit orifice et ledit manchon et un maintien en position efficace de ce dernier. Un adhésif ou de la colle peut éventuellement être ajouté entre la surface interne de l'orifice 6 et la surface externe du manchon 61 de manière à sécuriser davantage le maintien en position.

La figure 2 illustre le cas où le cordon tubulaire 4 est directement connecté au brassard 2, ce cordon assurant une liaison directe entre le mano-poire MP et ledit brassard (figure 4). Dans ce cas, l'orifice femelle 6 du brassard 2 est aménagé dans une protubérance 212a, 212b, ledit orifice femelle étant en communication fluidique avec la poche gonflable dudit brassard 2. L'orifice femelle 6 débouche dans un conduit 60. Cet orifice femelle 6 peut éventuellement être pourvu du manchon cylindrique de chemisage 61 décrit précédemment, ou être dépourvu de ce manchon. Dans ce dernier cas, la paroi interne cylindrique de l'orifice femelle 6 est directement adaptée à l'embout mâle externe 51, 71, et plus particulièrement à leur structure de joints.

Comme cela apparaît sur les figures 4 et 6, l'entrée de l'orifice femelle 6 est préférentiellement disposée de manière à ce que son axe Y soit parallèle à la surface de la pièce d'interface 21a, 21b, lequel axe Y est perpendiculaire à l'axe longitudinal X du brassard 2. La demanderesse a constaté que cette orientation permet un raccordement plus aisé du manomètre 5 ou du cordon 4.

La protubérance 212a, 212b est façonnée en saillie sur une pièce d'interface 21a, 21 b, laquelle pièce d'interface fait office d'interface entre la poche gonflable 25 et l'orifice femelle 6 du brassard 2. La protubérance 212a, 212b est avantageusement réalisée dans un matériau rigide, en plastique ou en métal. Elle pourrait toutefois être réalisée dans un matériau plastique semi-rigide ou souple.

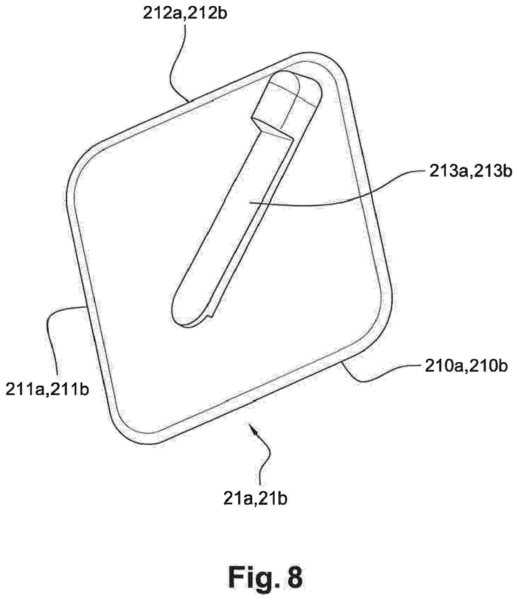

En se rapportant à la figure 8, la pièce d'interface 21 a, 21 b présente une face arrière 210a, 210b en contact avec le brassard 2 et une face avant 211a, 211b sur laquelle fait saillie la protubérance 212a, 212b. Sur la figure 8, elle a la forme d'un losange dont les côtés ont par exemple une longueur comprise entre 2 cm et 10 cm. Son épaisseur peut être comprise entre 1 mm et 20 mm.

La figure 9 illustre le cas où le brassard 2 est constitué d'une unique pièce dont la double paroi 23, 24 délimite la poche gonflable 25. Dans ce cas, la protubérance 212a et la pièce d'interface 21a forment une pièce monobloc, obtenue par exemple par moulage ou injection plastique. Cette pièce monobloc est préférentiellement réalisée dans un matériau thermoplastique, rigide ou semi-rigide, de manière à faciliter la soudure, mais elle peut être fabriquée dans un autre matériau rigide tel qu'un plastique ou un métal. La face arrière 210a de la pièce d'interface 21a est directement solidarisée sur une des parois paroi 23 du brassard 2, par exemple par un procédé de soudure ou par collage. Le conduit 60 débouche au niveau de la face arrière 210a de la pièce d'interface 21a, lequel conduit est positionné en vis-à-vis d'une ouverture 230 réalisée sur la paroi 23 de manière à assurer une communication fluidique entre l'orifice 6 et la poche gonflable 25.

La figure 10 illustre le cas où le brassard 2 est formé par deux parois 23, 24 non imperméables délimitant une poche dans laquelle est logée la poche gonflable 25 imperméable. Dans ce cas, la protubérance 212b et la pièce d'interface 21b peuvent être deux pièces distinctes. La protubérance 212b est préférentiellement réalisée dans un matériau thermoplastique, rigide ou semi-rigide, ou dans un autre matériau rigide tel qu'un plastique ou un métal. La pièce d'interface 21b peut être réalisée dans un matériau souple tel qu'un thermoplastique, ou en cuir ou dans un matériau rigide tel qu'un plastique ou un métal. La face arrière 210b de la pièce d'interface 21 b est solidarisée sur une des parois 23 du brassard 2, par exemple par collage ou par couture. La protubérance 212b est solidarisée sur la face avant 211b de la pièce d'interface 21b, par exemple par collage, par couture ou au moyen de rivets. Le conduit 60 débouche au niveau de la face avant 211b de la pièce d'interface 21 b dans laquelle est aménagée une ouverture, laquelle ouverture est positionnée en vis-à-vis d'une autre ouverture réalisée sur la paroi 23. La poche gonflable 25 est pourvue d'un embout 250 qui passe au travers de ces deux ouvertures pour se loger dans le conduit 60. On obtient ainsi une communication fluidique entre l'orifice 6 et la poche 25.

Selon une caractéristique avantageuse de l'invention, un aménagement en relief 213a, 213b, en forme de semelle, est installé devant l'orifice femelle 6 et dans l'axe Y de ce dernier. Cette semelle 213a, 213b permet de guider en position l'embout externe 71 du cordon 4 ou l'embout externe 51 du manomètre 5 (figure 7). Cette semelle 213a, 213b permet également de rigidifier et de renforcer la pièce d'interface 21 a, 21 b. Sa longueur est par exemple comprise entre 1 cm et 5 cm, sa largeur correspond préférentiellement à celle de la protubérance 212a, 212b, et son épaisseur est par exemple comprise entre 2 mm et 1 cm. Cette semelle 213a, 213b peut présenter une concavité dans l'axe Y de manière à faciliter le guidage de l'embout 71, 51.

La semelle 213a, 213b et la protubérance 212a, 212b peuvent être réalisées en une pièce unique ou en deux pièces distinctes. Elles peuvent faire partie intégrante de la pièce d'interface 21a, 21b et être réalisées au moment de sa fabrication. La semelle 213a, 213b peut également être rattachée à la pièce d'interface 21a, 21b ultérieurement grâce à des procédés de soudage, de collage, de rivetage, ou encore par couture. Le matériau utilisé pour la semelle 213a, 213b est préférentiellement le même que celui de la protubérance 212a, 212b. Elle peut toutefois être réalisée dans un autre matériau tel que le plastique, le métal, le cuir, etc.

Sur la figure 7, le manomètre 5 possède également un orifice femelle de connexion 6. Cet orifice est situé dans la partie inférieure du manomètre, à l'opposé de l'embout mâle externe 51, ledit orifice et ledit embout étant coaxiaux. L'orifice 6 est aménagé dans la bordure latérale 500 du cadran, perpendiculairement à l'axe Z dudit cadran. La poire de gonflage 3 et le mano-poire MP comportent également chacun un orifice femelle de connexion 6.

L'utilisation du système objet de l'invention va maintenant être décrite en détail. Le médecin a à sa disposition : le brassard 2, un seul cordon 4, le manomètre 5, la poire de gonflage 3, et le mano-poire MP.

Lorsque le médecin a besoin d'utiliser le mano-poire (figures 1, 2 et 4), il lui suffit de placer un brassard 2 de taille adaptée autour du bras du patient. Il connecte ensuite un des embouts mâles 71 du cordon 4 dans l'orifice femelle de connexion 6 du brassard 2. L'autre embout mâle 71 du cordon 4 est connecté dans l'orifice femelle de connexion 6 du mano-poire.

Lorsque le médecin a besoin d'utiliser un mano-brassard (figures 6 et 7), après avoir installé un brassard 2 de taille adaptée autour du bras du patient, il connecte l'embout mâle 51 du manomètre 5 dans l'orifice femelle de connexion 6 du brassard 2. Il connecte ensuite un des embouts mâles 71 du cordon 4 dans l'orifice femelle de connexion 6 du manomètre 5. L'autre embout mâle 71 du cordon 4 est connecté dans l'orifice femelle de connexion 6 de la poire de gonflage 3. Le cordon 4 assure ainsi une liaison directe entre la poire 3 et le manomètre 5.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de l'esprit et de la portée de l'invention. En particulier :
- Le brassard 2 peut être installé autour d'un membre approprié du patient tel que : bras, poignet, jambe, etc.
- Des moyens de gonflage 3 plus complexes qu'une poire peuvent être utilisés, comme par exemple une pompe à piston ou encore une pompe à engrenage.
- L'embout interne 72, l'embout externe 71 et la collerette 75 peuvent être fabriqués séparément et assemblés entre eux ultérieurement grâce à un procédé de soudure ou de collage par exemple.
- La pièce d'interface 21 a, 21 b n'est pas forcément en forme de losange, elle peut être de n'importe quelle forme, comme par exemple un carré, un cercle, une ellipse, etc.,
- La section du cylindre composant l'embout mâle externe 71, 51 n'est pas forcément circulaire, elle peut par exemple être carrée, rectangulaire ou encore elliptique,
- Le brassard 2 ne comporte pas forcément de pièce d'interface 21 a, 21b. L'orifice femelle 6 peut, par exemple, être agencé directement dans la poche gonflable 25. La protubérance 212a, 212b peut également être rattachée directement à la poche gonflable 25.
- Le cordon 4 pourrait comporter un orifice femelle de connexion à chaque extrémité ; le brassard comporter un embout mâle ; la poire de gonflage comporter un embout mâle pour accueillir un orifice femelle de connexion situé sur une des extrémités du cordon ; le mano-poire comporter un embout mâle pour accueillir un orifice femelle de connexion situé sur une des extrémités du cordon ; le manomètre comporter un orifice femelle de connexion pour se connecter sur un embout mâle du brassard et un embout mâle pour accueillir un orifice femelle situé sur une des extrémités du cordon.

## Revendications

1. Système de sphygmomanomètre (1) comprenant :
- un brassard gonflable (2) adapté pour être placé sur le membre d'un patient à examiner et/ou à surveiller, lequel brassard comporte une poche gonflable (25),
- une poire de gonflage (3) associée à un cordon tubulaire souple (4) pour assurer le gonflage du brassard (2),
- un manomètre pour mesurer la pression artérielle, lequel manomètre est soit associé à la poire de gonflage (3) pour former un mano-poire (MP), soit associé directement au brassard (2) pour former un mano-brassard (MB), **caractérisé en ce que** des moyens de connexion sont adaptés pour rendre interchangeable la configuration du système (1), lesquels moyens de connexion sont constitués d'aménagements en forme d'embouts mâles (51, 71) et orifices femelles (6), du type assemblage rapide, qui sont répartis :
- sur le brassard (2),
- sur le manomètre (5),
- sur la poire de gonflage (3),
- le mano-poire (MP),
- sur chaque extrémité (4a, 4b) du cordon tubulaire (4), de sorte que ledit cordon soit adapté pour assurer :
∘ d'une part, une liaison directe entre le mano-poire (MP) et le brassard (2), lorsque le manomètre (5) est connecté sur la poire de gonflage (3) pour former ledit mano-poire (MP),
∘ et d'autre part, une liaison directe entre la poire de gonflage (3) et le manomètre (5) lorsque ledit manomètre est connecté sur le brassard (2) pour former le mano-brassard (MB).

2. Système selon la revendication 1, **caractérisé en ce que** :
- le cordon (4) comporte un embout mâle (71) à chaque extrémité (4a, 4b),
- le brassard (2) comporte un orifice femelle de connexion (6),
- la poire de gonflage (3) comporte un orifice femelle de connexion (6) pour accueillir un embout mâle (71) situé sur une des extrémités (4a, 4b) du cordon (4),
- le mano-poire (MP) comporte un orifice femelle de connexion (6) pour accueillir un embout mâle (71) situé sur une des extrémités (4a, 4b) du cordon (4),
- le manomètre (5) comporte un embout mâle (51) pour se connecter sur l'orifice femelle de connexion (6) du brassard (2) et un orifice femelle (6) pour accueillir un embout mâle (71) situé sur une des extrémités (4a, 4b) du cordon (4).

3. Système selon l'une des revendications 1 ou 2, **caractérisé en ce que** l'orifice femelle de connexion (6) du brassard (2) est aménagé dans une protubérance (212a, 212b), ledit orifice femelle étant en communication fluidique avec la poche gonflable dudit brassard (2).

4. Système selon la revendication 3, **caractérisé en ce que** la protubérance (212a, 212b) est façonnée en saillie sur une pièce d'interface (21a, 21 b), laquelle pièce d'interface fait office d'interface entre la poche gonflable et l'orifice femelle (6) du brassard (2).

5. Système selon la revendication 4, **caractérisé en ce que** la pièce d'interface (21a) est soudée sur le brassard (2).

6. Système selon l'une des revendications 4 ou 5, **caractérisé en ce que** l'axe (Y) de l'orifice femelle de connexion (6) du brassard (2) est parallèle à la surface de la pièce d'interface (21a, 21b), ledit axe étant perpendiculaire à l'axe général longitudinal (X) dudit brassard (2).

7. Système selon l'une des revendications 3 à 6, **caractérisé en ce qu'**un aménagement en relief (213a, 213b), en forme de semelle, est installé devant l'orifice femelle de connexion (6) du brassard (2) et dans l'axe (Y) dudit orifice, laquelle semelle (213a, 213b) fait office de renfort et de guide pour la mise en place de l'embout mâle (51, 71) dans ledit orifice femelle (6).

8. Système selon l'une des revendications 1 à 7, **caractérisé en ce qu'**une structure de joint (73) est installée au niveau des embouts mâles (51, 71), l'entrée des orifices femelles (6) comportant un chemisage en forme de manchon cylindrique (61), ledit manchon étant adapté à ladite structure de joints (73).

9. Système selon l'une des revendications 1 à 8, **caractérisé en ce que** chaque embout mâle (51, 71) comporte plusieurs joints toriques (73) espacés longitudinalement.

10. Système de sphygmomanomètre (1) selon la revendication 9, **caractérisé en ce que** au moins deux des joints toriques (73) sont réalisés dans des matériaux différents.

11. Système selon l'une des revendications 1 à 10, **caractérisé en ce que** chaque moyen de connexion (7) du cordon tubulaire (4) comporte un embout mâle externe (71) et un embout mâle interne (72) séparés par une collerette (75), l'embout externe (71) étant muni d'au moins un joint torique d'étanchéité (73) et l'embout interne (72) étant solidarisé avec ledit cordon (4), lequel embout interne (72) comporte au moins un collet d'accroche (74) coopérant avec la paroi interne dudit cordon tubulaire (4).

12. Système selon l'une des revendications 8 à 11, **caractérisé en ce que** les embouts mâles (51, 71) sont en forme de cylindre dont le diamètre externe est ajusté au diamètre interne du manchon cylindrique (61).
